# EUROPÄISCHE PATENTANMELDUNG

(11) **EP 1 669 099 A1**
(43) Veröffentlichungstag der Anmeldung: **14.06.2006**
(21) Anmeldenummer: 04029128.8
(22) Anmeldetag: 09.12.2004
(51) Int. Cl.: A61M 25/10

(54) **Katheteranordnung für die medikamentöse Behandlung von Blutgefässen**

(71) Anmelder: Acrostak Corp., 8409 Winterthur (CH)
(72) Erfinder: Stahl, Laurent, 8404 Winterthur (CH); Berger, Erwin, 9506 Stettfurt (CH)
(74) Vertreter: Weiss, Wolfgang

(57) **Zusammenfassung**

Die Erfindung betrifft eine Katheteranordnung 1 für die medikamentöse Behandlung von Blutgefässen 2 mit mindestens einem Flanken 3, 4 aufweisenden Ballon 6, 8, wobei die Flanke 3, 4 einen Fortsatz 5 aufweisen und sowohl die Flanken 3,4 als auch der Fortsatz 5 perforiert sein kann.

## Beschreibung

Die Erfindung betrifft eine Katheteranordnung für die medikamentöse Behandlung von Blutgefässen nach den Merkmalen des Oberbegriffs von Anspruch 1.

Derartige Katheteranordnungen dienen dazu, ein Medikament an den erkrankten Bereich eines Blutgefässes zu verabreichen mit dem Ziel, eine optimale Wirkung zu erreichen. Dieser Idee der lokalen Applikation liegt der Gedanke zugrunde mit einer hohen lokalen Wirkstoffkonzentration und geringeren systemischen Nebenwirkungen effektivere Ergebnisse zu erzielen.

Zum Stand der Technik gehören die Anwendung von mit einem Medikament beschichtete Stents. Weiterhin sind Ballonsysteme mit perforierten Bereichen bekannt.

In der US 5,232,444 ist eine solche Katheteranordnung beschrieben. Die Anordnung weist einen aufblasbaren Ballon auf, wobei der Mittelbereich des Ballons zur Abgabe eines Medikamentes an die kranke Stelle des Blutgefässes perforiert ist.

Alle diese bekannten Applikationskatheter sind mit Nachteilen verbunden. Die Katheteranordnungen weisen Grenzen im Hinblick auf die Effizienz der kathetergestützten lokalen Therapie durch einen generell niedrigen Wirkstofftransfer und ein schnelles Auswaschen der Substanz aus der Gefässwand auf. Da die physikalischen Möglichkeiten, einen Wirkstoff mittels Katheter in die Gefässwand einzubringen, limitiert sind und sich zwischen den beiden Möglichkeiten der passiven Applikation (Gefässwand wird im Wirkstoff gebadet) einerseits und der aktiven Applikation (Wirkstoff wird mit maximal möglichem Druck in die Gefässwand eingebracht) bewegen, wurde nach einer geeigneten Katheteranordnung gesucht, die es erlaubt, sich kontinuierlich zwischen den beiden aktiven und passiven Möglichkeiten zu bewegen. Im übrigen ist bei diesen bekannten Katheteranordnungen eine vollständige Entleerung des Behandlungsraumes nicht gewährleistet mit der Folge, dass das Medikament teilweise vom Blut weggespült wird und vom Körper abgebaut werden muss. Es entstehen dadurch nicht gewünschte systemische Verbreiterungen des Medikamentes im Körper.

Die mit der vorliegenden Erfindung verbundene Aufgabe liegt somit darin, eine Katheteranordnung zur Verabreichung eines Medikamentes in einen erkrankten Bereich eines Blutgefässes vorzuschlagen, bei dem längere Applikationszeiten unter Vermeidung eines Gefässtraumas möglich sind.

Diese Aufgabe wird erfindungsgemäss durch die Merkmale des Kennzeichens von Anspruch 1 gelöst.

Kern der Erfindung ist es, eine Einströmrichtung des jeweiligen Medikamentes in den Behandlungsraum so führen, dass das Medikament nicht direkt, d.h. mit hohem Druck, auf die erkrankte Stelle des Blutgefässes auftrifft.

Mit dieser erfindungsgemässen Massnahme wird erreicht, dass ein Wirkstoff in hoher Konzentration in homogener und nicht gefässtraumatisierender Weise über einen definierten Zeitraum vor Ort abgegeben werden kann. Beispielsweise ist für die Abgabe von Paclitaxel diese Tatsache entscheidend. Paclitaxel hinterlässt seine zelluläre Wirkung, nämlich eine Änderung des Zytoskeletts, auch dann noch, wenn die Substanz selbst schon längst ausgewaschen ist. Bei der Anwendung der erfindungsgemäsen Katheteranordnung werden die schädlichen Nebenwirkungen vermieden, zumindest reduziert. In diesem Zusammenhang werden beispielhaft folgende weitere für dieses erfindungsgemässe Verfahren geeignete Substanzen genannt: Sirolimus, Everolimus und Stammzellen.

Zur Einführung des Medikamentes in den Behandlungsraum an die erkrankte Stelle sind vorzugsweise Perforationen in den Flanken und/oder der Fortsätze der Ballone vorgesehen. Die Perforationen können im Minimum aus einem Loch oder aus sehr vielen Löchern bestehen.

Mindestens ein Ballon ist entfalt - und aufblasbar. Die Katheteranordnung weist mindestens zwei benachbarte Ballone in Richtung des Blutgefässes auf und sind im aufgeblasenen Zustand durch einen Zwischenraum getrennt.

Mit dem Medikament wird auch gleichzeitig der distale und proximale Ballon in folgender Weise entfaltet und aufgeblasen:

Das Medikament wird durch einen Schaft in die Katheteranordnung eingespritzt. Dadurch entfaltet sich zuerst der Ballon in seine Form und entwickelt durch seine hantelförmige Ausgestaltung (distaler und proximaler Ballon) eine dichtende Wirkung auf beide Seiten des zu behandelnden Bereiches. Das der Ballon sich zuerst entfaltet erreicht man technisch dadurch, dass der Gesamtquerschnitt der Perforationen in der/den Flanken genügend klein ist. Es soll damit vermieden werden, dass das Medikament ohne Entfaltung des Ballons aus den Perforationen heraustritt. Durch den erfindungsgemässen Fortsatz werden beispieslweise die perforierten Ballonflanken beim Entfalten der Ballone nahezu senkrecht zur Strömungsrichtung des Blutgefässes ausgerichtet. Das Entleeren des Applikationsraumes geschieht durch Anwendung von Unterdruck. Das Medikament wird durch die Perforationen wieder zurückgezogen. Dabei muss das Volumen des Applikationsraumes genügend klein gegenüber dem Gesamtvolumen des Katheters gewählt werden.

In vorteilhafter Weise beträgt der Winkel α -gebildet aus Richtung F der Flanke und der Senkrechten S zur Strömungsrichtung des Blutgefässes - 0° bis 30°.

Die Erfindung wird im folgenden mittels zweier Ausführungsbeispiele beschrieben. Es zeigen:
Fig. 1 eine Katheteranordnung mit perforierter Flanke
Fig.2 eine Katheteranordnung mit perforiertem Fortsatz

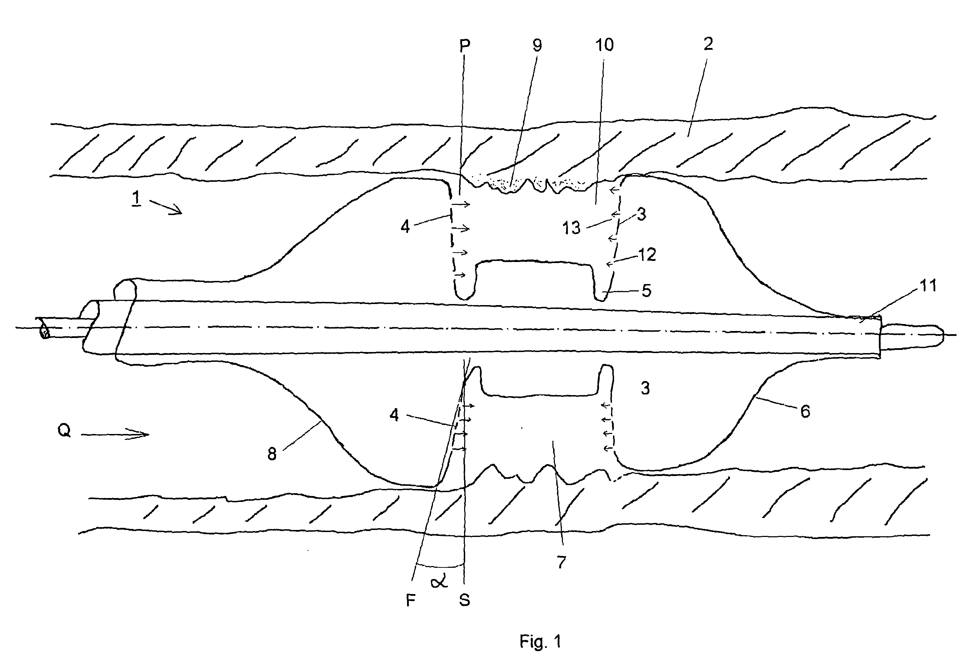

Die Katheteranordnung 1 mit dem Führungsdraht 11 in Figur 1 ist aufgeblasen in das Blutgefäss 2 eingeführt und positioniert gezeigt. Die Katheteranordnung 1 weist zwei durch den Zwischenraum 7 getrennte Ballone 6 und 8 auf. Im Zwischenraum 7 ist der zu behandelnde Bereich 9 schematisch dargestellt. Der Zwischenraum 7 ist durch die Flanken 3 und 4 der Ballone 6 und 8 abgegrenzt. Um den Winkel α, der aus der Richtung der Flanken 3 bzw. 4 und der Senkrechten S zur Strömungsrichtung Q des Blutes gebildet wird, möglichst klein zuhalten, ist jeweils ein Fortsatz 5 bei den Flanken 3 und 4 vorgesehen. Die Flanken 3 und 4 sind für die Zugabe des Medikamentes 10 perforiert. Die Flanken 3 und 4 sind mit Löchern 13 aufweisenden Perforationen versehen.

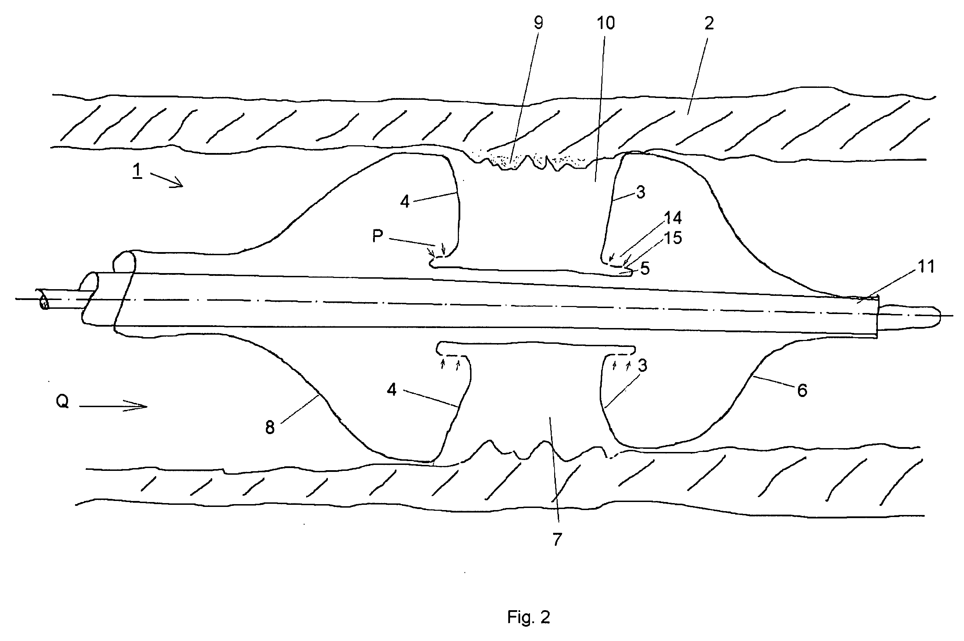

Bei der Figur 2 weisen die Flanken 3 und 4 keine Perforationen auf. Die Perforationen 14 mit den Löchern 15 sind in dem Fortsatz 5 angeordnet. Der Fortsatz 5 erstreckt sich parallel zur Blutgefässströmung Q.

Die erfindungsgemässe Katheteranordnung kann auch zur Behandlung anderer Organsysteme, wie Trachea, Bronchien, Speiseröhre, ableitende Harnwege oder Gallenkanäle mittels spezifischer Medikamente verwendet werden.
Die mit der Erfindung verbundenen Vorteile liegen insbesondere darin, dass eine möglichst senkrechte Flankenrichtung infolge des Fortsatzs erreicht wird.

### Bezugszeichenliste

- 1: Katheteranordnung
- 2: Blutgefäss
- 3: Flanke
- 4: Flanke
- 5: Fortsatz
- 6: Ballon
- 7: Zwischenraum
- 8: Ballon
- 9: Zu behandelnder Bereich
- 10: Medikament
- 11: Draht
- 12: Perforation
- 13: Loch
- 14: Perforation
- 15: Loch

- α: Winkel
- F: Flankenrichtung
- S: Richtung der Senkrechten
- P: Einströmrichtung
- Q: Blutgefässströmung

## Patentansprüche

1. Katheteranordnung (1) für die medikamentöse Behandlung von Blutgefässen (2) mit mindestens einem Flanken (3, 4) aufweisenden Ballon (6, 8), **dadurch gekennzeichnet, dass** eine Flanke (3, 4) einen Fortsatz (5) aufweist.

2. Katheteranordnung nach Anspruch 1, **dadurch gekennzeichnet, dass** der Fortsatz (5) im Querschnitt U-förmig ausgebildet ist.

3. Katheteranordnung nach Anspruch 1 oder 2, **dadurch gekennzeichnet, dass** der Fortsatz (5) senkrecht zur Strömungsrichtung Q des Blutgefässes (2) verläuft.

4. Katheteranordnung nach mindestens einem der Ansprüche 1, 2 oder 3, **dadurch gekennzeichnet, dass** der Fortsatz (5) parallel zur Strömungsrichtung Q des Blutgefässes (2) verläuft.

5. Katheteranordnung nach mindestens einem der vorangehenden Ansprüche , **dadurch gekennzeichnet, dass** der Fortsatz (5) eine Perforierung (14) mit mindestens einem Loch (15) aufweist.

6. Katheteranordnung nach mindestens einem der vorangehenden Ansprüche , **dadurch gekennzeichnet, dass** die Flanke (3, 4) eine Perforierung (12) mit mindestens einem Loch (13) aufweist.

7. Katheteranordnung nach mindestens einem der vorangehenden Ansprüche , **dadurch gekennzeichnet, dass** der Winkel α -gebildet aus Richtung F der Flanke (3, 4) und der Senkrechten S zur Strömungsrichtung des Blutgefässes (2) - 0° bis 30° beträgt.

8. Katheteranordnung nach mindestens einem der vorangehenden Ansprüche , **dadurch gekennzeichnet, dass** mindestens ein Ballon (6,8) entfalt - und aufblasbar ist.

9. Katheteranordnung nach mindestens einem der vorangehenden Ansprüche , **dadurch gekennzeichnet, dass** die Katheteranordnung (1) zwei Ballone (6, 8) aufweist, wobei die Ballone (6, 8) benachbart in Richtung des Blutgefässes (2) angeordnet und im aufgeblasenen Zustand durch einen Zwischenraum (7) getrennt sind.
